# EUROPEAN PATENT SPECIFICATION

(11) **EP 0 570 443 B1**
(45) Date of publication and mention of the grant of the patent: **29.11.1995**
(21) Application number: 92904156.4
(22) Date of filing: 07.02.1992
(51) Int. Cl.: C07C 217/10

(54) **AMINOALCOHOLS USEFUL AS OPTICAL RESOLVING AGENTS**
AMINOALKOHOLE ALS REAGENZIEN ZUR OPTISCHEN AUFLÖSUNG
AMINOALCOOLS UTILES COMME AGENTS OPTIQUES RESOLVANTS

(30) Priority: 08.02.1991 FR 9101451
(43) Date of publication of application: 24.11.1993
(73) Proprietor: SMITHKLINE BEECHAM LABORATOIRES PHARMACEUTIQUES, 92731 Nanterre Cédex (FR)
(72) Inventor: BROWN, Eric Faculté des Sciences, F-72100 Le Mans (FR); TOUET, Jöel Faculté des Sciences, F-72100 Le Mans (FR); LE GOFF, Jean-Pierre, BP2 F-53101 Mayenne Cédex (FR)
(74) Representative: Rutter, Keith, Dr.
(86) International application number: EP9200300
(87) International publication number: WO9213823

(56) References cited:
- EP-A- 0 000 745
- FR-A- 2 107 926
- Journal of Organic Chemistry, vol. 8, January 1943, K. JOHNSON et al.: "The utilization of aliphatic nitro compounds. V. Reduction of nitro alcohols and nitro glycols to the corresponding amines", pages 7-9, see whole document(cited in the application)

## Description

The present invention relates to novel compounds which are useful resolving agents for certain racemates, especially for DL-N-acetyl-4-hydroxyphenylglycine (hereinafter DL-N-acetyl-4-HPG) or DL-N-haloacetyl-4-HPG. The invention also relates to a process for effecting resolution of such racemates, especially for resolving DL-N-acetyl-4-HPG and DL-N-haloacetyl-4-HPG.

US Patent No. 3869505 discloses the preparation of DL-N-acetyl-4-HPG, and its use in resolving DL-2-(4-hydroxyphenyl)-glycine (hereinafter DL-2-4-HPG) into its optical isomers. The D(-) form of the latter is particularly useful in the preparation of penicillin and cephalosporin derivatives. European Patent Application, Publication No. 0000745 discloses the optical resolution of certain amino acids by the use of optically active 2 - aminobutanol.

A group of novel compounds has now been discovered which has particular utility in the resolution of DL-N-acetyl-4-HPG or DL-N-haloacetyl-4-HPG into their (D-) forms, each of which can then be converted in known manner to the (D-) form of DL-2-4-HPG. These novel compounds may also be effectively used in the resolution of other acidic racemates.

According to the present invention, there is provided a compound of formula (I), or a salt or solvate thereof, 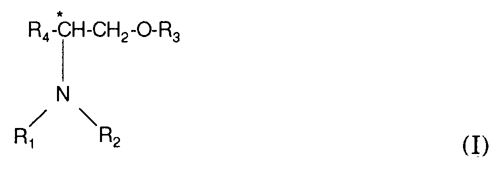
in which R₁, R₂, R₃ and R₄ are as defined in claim 1.

Suitably, R₁ and R₂ each independently represent hydrogen or alkyl. Favourably, R₁ is hydrogen. Favourably, R₂ is hydrogen. Preferably, R₁ and R₂ each represent hydrogen.

Suitably, R₃ is an optionally substituted aralkyl group, such as an optionally substituted phenyl C₁₋₆ alkyl group, for example an optionally substituted benzyl group. It is preferred if the aralkyl group is substituted in the aryl moiety.

Suitable substituted aralkyl groups include mono-halo and bis-halo substituted aralkyls, in particular 4-halo and 2,4-halo substituted aralkyl groups.

Examples of substituted aralkyl groups include 4-chlorobenzyl, 4-bromobenzyl and 2,4-dichlorobenzyl.

An example of an unsubstituted aralkyl group is a benzyl group.

Suitably, R₄ is an alkyl group; especially a C₁₋₆ alkyl group. Preferably, R₄ is an ethyl group.

One preferred sub-group of compounds of formula (I) is a compound of formula (II), or a salt or solvate thereof, 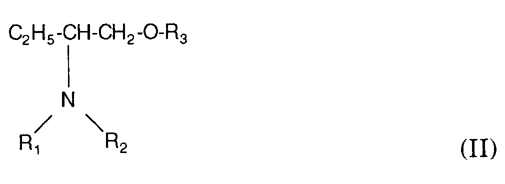
in which each of R₁ and R₂ is hydrogen, C₁₋₆ alkyl, optionally substituted phenyl or optionally substituted phenyl C₁₋₆ alkyl; and R₃ is optionally substituted phenyl or optionally substituted phenyl C₁₋₆ alkyl.

A particularly preferred sub-group of the compounds of formula (I) has the formula (III); 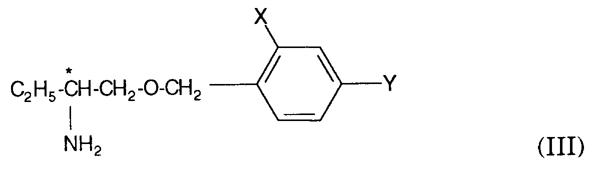
in which each of X and Y represents halogen, preferably chlorine.

The carbon atoms in the above formulae annotated with an asterisk (*) are chiral carbons. The compounds of formula (I) (and thus (II) and (III)) can therefore exist in one of two enantiomeric forms. The present invention extends to single isomers, including enantiomers, of formula (I) as well as to mixtures thereof; including racemates. Preferably the compounds of formula (I) are in the form of an optically pure enantiomer.

Thus in one aspect the present invention provides a compound of formula (I), or a salt or solvate thereof, in the form of a single enantiomer. One form is the (R) enantiomer. One form is the (S) enantiomer.

Specific compounds of the invention are;
(R)-(-)-2-amino-1-(2,4-dichlorobenzyloxy)butane;
(R)-(-)-2-amino-1-benzyloxybutane;
(R)-(-)-2-amino-1-(4-chlorobenzyloxy)butane;
(R)-(-)-2-amino-1-(4-bromobenzyloxy)butane; and
S-(+)-2-amino-1-(4-chlorobenzyloxy)butane.

Examples of salts of the compounds of the invention are acid addition salts with conventional acids such as hydrochloric or maleic acids.

An example of a solvate is a hydrate.

When used herein the term 'alkyl' (whether used alone or when used as part of another group for example as in an 'aralkyl' group) includes straight and branched chain alkyl groups, containing from 1 to 12 carbon atoms, suitably 1 to 6 carbon atoms, for example methyl, ethyl, propyl or butyl.

When used herein the term 'aryl' (whether used alone or when used as part of other groups for example as in an 'aralkyl' group) includes phenyl and naphthyl optionally substituted with up to five, preferably up to three, groups selected from halogen, alkyl, phenyl, alkoxy, hydroxy, amino, nitro, alkoxycarbonyl, alkoxycarbonylalkyl,alkylcarbonyloxy, or alkylcarbonyl groups.

A preferred aryl group is a phenyl group.

A preferred substituent for an aryl group is halo.

The compounds of formula (I) may be prepared by reacting a compound of formula (IV) 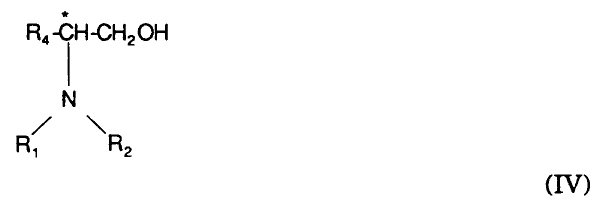
in which R₁, R₂ and R₄ are as defined in formula (I), with a compound of formula (V):

Hal-R₃ (V)

in which Hal is halogen, preferably chlorine, and R₃ is as defined in formula (I); and thereafter, if required, preparing a salt or solvate of the compound of formula (I).

Preferably, the compound of formula (IV) is treated with a base, such as sodium hydride prior to reaction with compound (V).

Most preferably, for the preparation of single enantiomers of the compounds of formula (I), the compound of formula (IV) is also in the form of a single enantiomer: The chirality of the asterisked carbon in the compound of formula (IV) being the same as that in the required compound of formula (I).

The compounds of formulae (IV) and (V) are known commercially available compounds, or can be prepared from known compounds by known methods, e.g. as in USP 2174242 or J. Org. Chem. 8, 7, (1943).

The invention further provides a process for the resolution of an acidic racemate into individual enantiomers, which process comprises:
(i) preparing diastereomeric salts from the acidic racemate and a single enantiomer of a compound of formula (I) or a salt or solvate thereof;
(ii) separating the diastereomeric salts; and
(iii) preparing an individual enantiomer of the acidic racemate from a diastereomeric salt.

The preparation of the diastereomeric salts may be carried out in any suitable solvent, generally an organic solvent such as isopropyl alcohol, at any convenient temperature, but generally at an elevated temperature.

The separation of the diastereomeric salts may be carried out by use of any conventional method, such as fractional crystallization. One preferred method involves seeding the mixture with the required salt to facilitate separation and especially to facilitate crystallizastion.

An enantiomer may be prepared from a diastereomeric salt by any conventional means. One suitable method involves treating the salt with a base and then separating the individual enantiomer of the starting racemate and the isomer of the compound of formula (I), for example by solvent extraction.

Generally, the isomer of the compound of formula (I) is recovered and then reused in the process.

The individual enantiomer of the starting racemate may be further purified by recrystallisation, if and as required.

Generally, the acidic racemate is an amino acid racemate, favourably an N-acyl amino acid.

One preferred racemate is DL-N-acetyl-4-hydroxyphenylglycine.

One preferred racemate is DL-N-haloacetyl-4-hydroxyphenylglycine.

Thus, in one preferred aspect the invention provides the preparation of DL-N-acetyl-4-HPG or DL-N-haloacetyl-4-HPG in optically active D(-) form, which comprises treating either compound, in solution, with a compound of formula (I), or a salt or solvate thereof, to prepare the mixture of diastereoisomeric salts, allowing the diastereoisomeric salt containing the optically active D(-) form (the D(-) containing salt) to precipitate out, treating the D(-) containing salt with base, and separating the precipitated D(-) form from the solution.

Preferably, the DL-starting material is dissolved in an organic solvent, such as ethanol, at elevated temperature, the single stereoisomer of a compound of a compound of formula (I), or a salt or solvate thereof, is added, and the solution allowed to cool to room temperature after seeding with pure D(-) containing salt which is then used to provide the required optically active D(-) form.

The pure D-(-)-N-acetyl-4-HPG may be converted to D(-)2-4-HPG by conventional deacylation procedures, for example those disclosed in 'Protective Groups in Organic Chemistry' by J.F.W. McOmie, Plenum Press, London and New York, 1973 or French Patent No. 2107926.

The invention is illustrated by the following Examples:

### Example 1

### Preparation of (R)-(-)-2-Amino-1-(2,4-dichlorobenzyloxy)butane

Pure (R)-(-)-2-amino-1-butanol (34.1g) is treated with sodium hydride (11g) in toluene (375ml) by refluxing for 4 hours. 2,4-Dichlorobenzylchloride (75g) is added to the reaction mixture and refluxed for a further 4 hours. The mixture is then cooled and 1M hydrochloric acid (900ml) is added.

The aqueous layer is separated and made alkaline by addition of 1M sodium hydroxide solution (600ml) Methylene dichloride (500ml) is added to the aqueous layer and the organic and aqueous phases are separated. The organic phase is concentrated and distilled in order to obtain the pure product. Yield 70%.

[a]²⁰_{D} = -12.5° (C 2.84 EtOH)
EbO,1 = 120°
IR _{νmax} (film): 3376 (N=H) et 1093 (C-O-C) cm⁻¹
NMR (CDCl₃) δ (ppm): 7.5 (3H.m): 4.6 (2H, s): 3.5 (2H. m): 3.0 (1H, m): 1.45 (4H, m): 1.0 (3H, t).

| Elemental microanalysis (as the hydrochloride) | | | | | | | | | |
|---|---|---|---|---|---|---|---|---|---|
| (C₁₁H₁₆Cl₃NO): | Calc. % | C | 46.42 | H | 5.67 | N | 4.92 | O | 5.62 |
| | Found % | | 46.60 | | 5.76 | | 5.01 | | 5.84 |

### Examples 2-4

The following compounds were then prepared using an analogous procedure to that described for the preparation of Example 1:

### Example 2

### R-(-)Amino-1-benzyloxybutane:

IR_{νmax} (film): 3400 (δNH): 1102 (C-O-C); 856 (NH) and 740; 699 (aryl) cm⁻¹
NMR (CDCl₃) δ (ppm): 7.33 (5H, s); 4.5 (2H, s); 3.3 (2H, m); 2.85 (1H, m); 1.3 (4H, m); 0.9 (3H, t).

| Elemental microanalysis (as the hydrochloride) | | | | | | | | | |
|---|---|---|---|---|---|---|---|---|---|
| (C₁₁H₁₈CINO): | Calc. %: | C | 61.24 | H | 8.40 | N | 6.59 | O | 7.41 |
| | Found % | | 61.02 | | 8.57 | | 6.65 | | 7.66 |

### Example 3

### R-(-)-2-Amino-1-(4-chlorobenzyloxy)butane:

IR_{νmax} (film): 3376 (N-H) and 1093 (C-O-C) cm⁻¹
NMR (CDCl₃) δ(ppm) 7.33 (4H, s); 4.5 (2H, s); 3.35 (2H, m); 2.9 (1H, m); 1.33 (4H, m); 0.9 (3H, t).

| Elemental microanalysis (as the hydrochloride) | | | | | | | |
|---|---|---|---|---|---|---|---|
| (C₁₁H₁₇Cl₂NO): | Calc % | C | 52.81 | H | 6.85 | N | 5.60 |
| | Found % | | 52.58 | | 6.68 | | 5.66 |

### Example 4

### R-(-)-2-Amino-1-(4-bromobenzyloxy)butane

IR_{νmax} (film): 3376 (N-H) and 1093 (C-O-C) cm⁻¹
NMR (CDCl₃) δ(ppm): 7.55 (2H. d. J=8.5 Hz): 7.25 (2H, d, J=8.5 Hz); 4.5 (2H, s); 3.35 (2H, m); 2.9 (1H, m); 1.33 (4H, m); 0.9 (3H, t).

| Elemental microanalysis (as the hydrochloride) | | | | | | | | | | | | | |
|---|---|---|---|---|---|---|---|---|---|---|---|---|---|
| (C₁₁H₁₇ClBrNO): | Calc.% | C | 44.84 | H | 5.81 | Cl | 12.03 | Br | 27.12 | N | 4.85 | O | 4.99 |
| | Found % | | 45.17 | | 5.72 | | 12.19 | | 27.15 | | 4.85 | | 5.43 |

### Example 5

### S(+)-2-Amino-1-(4-chlorobenzyloxy)butane

The title compound was prepared using an analogous procedure to that described in Example 1, starting from S(+)-2-amino-1-butanol.

IR_{νmax} (film); 3376 (N-H) and 1093 (C-O-C) cm⁻¹
NMR (CDCl₃) δ(ppm) 7.33 (4H, s); 4.5 (2H, s); 3.35 (2H, m); 2.9 (1H, m); 1.33 (4H, m); 0.9 (3H, t).

| Elemental microanalysis (as the hydrochloride) | | | | | | | | | |
|---|---|---|---|---|---|---|---|---|---|
| (C₁₁H₁₇Cl₂NO): | Calc. % | C | 52.81 | H | 6.85 | N | 5.60 | O | 6.40 |
| | Found % | | 53.25 | | 6.94 | | 5.55 | | 6.33 |

### Example 6

### Resolution of DL-N-acetyl-4-HPG

DL-N-acetyl-4-HPG is prepared according US Patent 3869505, and 50g of this are dissolved in isopropyl alcohol (1000ml) at elevated temperature.

(R)-(-)-2-amino-1-(2,4 dichlorobenzyloxy)-butane [(-)ADCBB] (60g) as prepared in Example 1, are added with stirring, the mixture is seeded with pure D-(-)-N-acetyl-4-HPG, (-)ADCBB salt and allowed to cool to room temperature. After standing at room temperature overnight, the precipitate formed is collected by filtration and washed with isopropyl alcohol. After drying, 50.9g of D-(-)-N-acetyl-4-HPG, (-)ADCBB salt are obtained. Yield = 93% [a]²⁴ = 89.6% (Cl EtOH 95°)
The salt is then dissolved in water by the addition of sodium hydroxide. The (-)ADCBB base is recovered by separation and distillation of the organic upper layer. The D-(-)N-acetyl-4-HPG is precipitated from the aqueous layer by neutralizing this layer with hydrochloric acid. After filtration and drying 44.1g of pure D-(-)-N-acetyl-4-HPG are obtained, yield = 95%.
[a]²⁰_{D} = -214.9° (Cl 1.02, EtOH 95°)
The resolving agent is recovered as the hydrochloride or free base, according to the medium used.

### Example 7

### Resolution of DL-N-chloroacetyl-4-HPG

According to the method of Example 6, the D-(-) form of the above was achieved in the indicated yields, using the following resolving agents

| | |
|---|---|
| (R)-(-)-2-amino-1-benzyloxybutane | yield = 75%. |
| (R)-(-)-2-amino-1-para-chlorobenzyloxybutane | yield = 53%. |
| (R)-(-)-2-amino-para-bromo-1-benzyloxybutane | yield = 61%. |
| (R)-(-)-2-amino-1-(2,4 dichlorobenzyloxy)butane | yield = 66%. |

## Claims

1. A compound of formula (I), or a salt or solvate thereof, 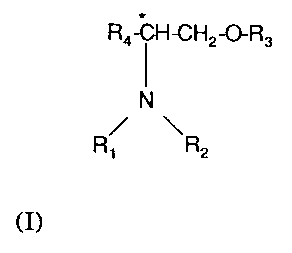 in which R₁ and R₂ each independently represents hydrogen, C₁₋₁₂-alkyl, optionally substituted aryl or aryl-C₁₋₁₂-alkyl optionally substituted in the aryl moiety; R₃ represents C₁₋₁₂-alkyl, optionally substituted aryl or aryl-C₁₋₁₂-alkyl optionally substituted in the aryl moiety and R₄ represents C₁₋₁₂-alkyl or optionally substituted aryl;
wherein any aryl group is a phenyl or naphthyl group optionally substituted with up to five groups selected from halogen, C₁₋₁₂-alkyl, phenyl, C₁₋₁₂-alkoxy, hydroxy, amino, nitro, C₁₋₁₂-alkoxycarbonyl, C₁₋₁₂-alkoxycarbonyl-C₁₋₁₂-alkyl, C₁₋₁₂-alkylcarbonyloxy or C₁₋₁₂-alkylcarbonyl.

2. A compound according to claim 1, wherein R₁ and R₂ each independently represent hydrogen or C₁₋₁₂-alkyl.

3. A compound according to claim 1 or claim 2, wherein R₁ and R₂ each represent hydrogen.

4. A compound according to any one of claims 1 to 3, wherein R₃ is a benzyl group.

5. A compound according to any one of claims 1 to 4, wherein R₄ is a C₁₋₁₂-alkyl group.

6. A compound according to claim 1 being of formula (II), or a salt or solvate thereof, 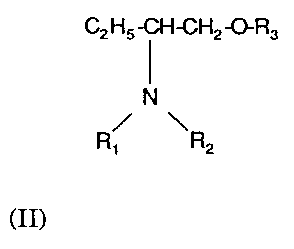 in which each of R₁ and R₂ is hydrogen, C₁₋₆ alkyl, optionally substituted phenyl or optionally substituted phenyl C₁₋₆ alkyl; and R₃ is optionally substituted phenyl or optionally substituted phenyl C₁₋₆ alkyl.

7. A compound of formula (I) according to claim 1, or a salt or solvate thereof, in the form of an optically pure enantiomer.

8. A compound according to claim 1, selected from the group consisting of:
(R)-(-)-2-amino-1-(2,4-dichlorobenzyloxy)butane;
(R)-(-)-2-amino-1-benzyloxybutane;
(R)-(-)-2-amino-1-(4-chlorobenzyloxy)butane;
(R)-(-)-2-amino-1-(4-bromobenzyloxy)butane; and
(S)-(+)-2-amino-1-(4-chlorobenzyloxy)butane; or a salt or solvate thereof.

9. A process for preparing a compound of formula (I) according to claim 1, which process comprises reacting a compound of formula (IV): 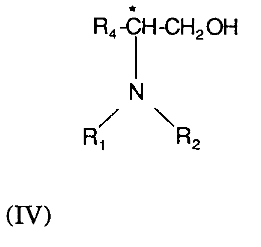 in which R₁, R₂ and R₄ are as defined in formula (I), with a compound of formula (V):
Hal-R₃ (V)
in which Hal is halogen, preferably chlorine, and R₃ is as defined in formula (I); and thereafter, if required, preparing a salt or solvate of the compound of formula (I).

10. A process for the resolution of an acidic racemate into individual enantiomers, which process comprises:
(i) preparing diastereomeric salts from the acidic racemate and a single enantiomer of a compound of formula (I) according to claim 1 or a salt or solvate thereof;
(ii) separating the diastereomeric salts; and
(iii) preparing an individual enantiomer of the acidic racemate from a diastereomeric salt.

11. A process according to claim 10, for the preparation of DL-N-acetyl-4-HPG or DL-N-haloacetyl-4-HPG in optically active D(-) form, which comprises treating either compound, in solution, with a compound of formula (I), or a salt or solvate thereof, to prepare the mixture of diastereoisomeric salts, allowing the diastereoisomeric salt containing the optically active D(-) form (the D(-) containing salt) to precipitate out, treating the D(-) containing salt with base, and separating the precipitated D(-) form from the solution.

## Patentansprüche

1. Verbindung der Formel (I), oder ein Salz oder Solvat davon, 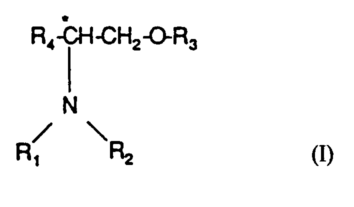 in der R₁ und R₂ jeweils unabhängig ein Wasserstoffatom, einen C₁₋₁₂-Alkyrest, eine gegebenenfalls substituierte Arylgruppe oder einen gegebenenfalls im Arylteil substituierten Aryl-C₁₋₁₂-Alkylrest darstellen, R₃ einen C₁₋₁₂-Alkyrest, einen gegebenenfalls substituierten Arylrest oder einen gegebenenfalls im Arylteil substituierten Aryl-C₁₋₁₂-Alkylrest darstellt und R₄ einen C₁₋₁₂-Alkylrest oder einen gegebenenfalls substituierten Arylrest darstellt;
wobei jede Arylgruppe eine Phenyl- oder Naphthylgruppe ist, die gegebenenfalls mit bis zu fünf Gruppen substituiert ist, ausgewählt aus Halogenatomen oder C₁₋₁₂-Alkyl-, Phenyl-, C₁₋₁₂-Alkoxy-, Hydroxy-, Amino-, Nitro-, C₁₋₁₂-Alkoxycarbonyl-, C₁₋₁₂-Alkoxycarbonyl-C₁₋₁₂-Alkyl-, C₁₋₁₂-Alkylcarbonyloxy- oder C₁₋₁₂-Alkylcarbonylresten.

2. Verbindung nach Anspruch 1, in der R₁ und R₂ jeweils unabhängig ein Wasserstoffatom oder einen C₁₋₁₂-Alkylrest darstellen.

3. Verbindung nach Anspruch 1 oder Anspruch 2, in der R₁ und R₂ jeweils ein Wasserstoffatom darstellen.

4. Verbindung nach einem der Ansprüche 1 bis 3, in der R₃ eine Benzylgruppe ist.

5. Verbindung nach einem der Ansprüche 1 bis 4, in der R₄ ein C₁₋₁₂-Alkylrest ist.

6. Verbindung nach Anspruch 1 der Formel (II), oder ein Salz oder Solvat davon, 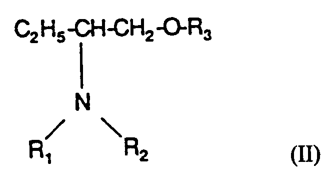 in der R₁ und R₂ jeweils ein Wasserstoffatom, einen C₁₋₆-Alkylrest, eine gegebenenfalls substituierte Phenylgruppe oder einen gegebenenfalls substituierten Phenyl-C₁₋₆-Alkylrest darstellen und R₃ eine gegebenenfalls substituierte Phenylgruppe oder einen gegebenenfalls substituierten Phenyl-C₁₋₆-Alkylrest darstellt.

7. Verbindung der Formel (I) nach Anspruch 1, oder ein Salz oder Solvat davon, in Form eines optisch reinen Enantiomeren.

8. Verbindung nach Anspruch 1, ausgewählt aus:
(R)-(-)-2-Amino-1-(2,4-dichlorbenzyloxy)butan,
(R)-(-)-2-Amino-1-benzyloxybutan,
(R)-(-)-2-Amino-1-(4-chlorbenzyloxy)butan,
(R)-(-)-2-Amino-1-(4-brombenzyloxy)butan und
(S)-(+)-2-Amino-1-(4-chlorbenzyloxy)butan, oder ein Salz oder Solvat davon.

9. Verfahren zur Herstellung einer Verbindung der Formel (I) nach Anspruch 1, welches die Umsetzung einer Verbindung der Formel (IV): 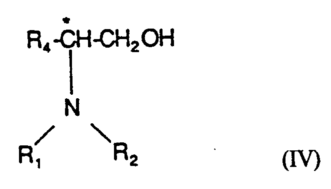 in der R₁, R₂ und R₄ die in Formel (I) angegebene Bedeutung haben, mit einer Verbindung der Formel (V):
Hal-R₃ (V)
in der Hal ein Halogenatom, vorzugsweise Chlor, ist, und R₃ die in Formel (I) angegebene Bedeutung hat; und anschließend, falls erforderlich, die Herstellung eines Salzes oder Solvats der Verbindung der Formel (I) umfaßt.

10. Verfahren zur Spaltung eines sauren Racemats in einzelne Enantiomere, das
(i) die Herstellung diastereomerer Salze aus dem sauren Racemat und einem einzelnen Enantiomer einer Verbindung der Formel (I) nach Anspruch 1 oder einem Salz oder Solvat davon,
(ii) die Trennung der diastereomeren Salze und
(iii) die Herstellung eines einzelnen Enantiomeren des sauren Racemats aus einem diastereomeren Salz umfaßt.

11. Verfahren nach Anspruch 10 zur Herstellung von DL-N-Acetyl-4-HPG oder DL-N-Haloacetyl-4-HPG in optisch aktiver D(-)-Form, das die Behandlung beider Verbindungen in Lösung mit einer Verbindung der Formel (I), oder einem Salz oder Solvat davon, um das Gemisch diastereoisomerer Salze herzustellen, das Ausfallenlassen des diastereoisomeren Salzes, das die optisch aktive D(-)-Form enthält (das D(-)-haltige Salz), die Behandlung des D(-)-haltigen Salzes mit einer Base und das Abtrennen der ausgefallenen D(-)-Form von der Lösung umfaßt.

## Revendications

1. Composé de formule (I), ou de ses sels ou produits de solvatation, 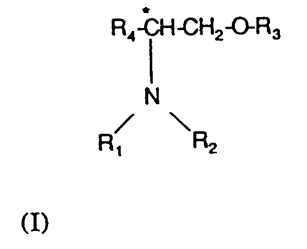 formule dans laquelle R₁ et R₂ représentent chacun, indépendamment, l'hydrogène, un groupe alkyle en C₁ à C₁₂, aryle facultativement substitué ou aryl-(alkyle en C₁ à C₁₂) facultativement substitué dans le groupement aryle ; R₃ représente un groupe alkyle en C₁ à C₁₂, aryle facultativement substitué ou aryl-(alkyle en C₁ a C₁₂) facultativement substitué dans le groupement aryle, et R₄ représente un groupe alkyle en C₁ à C₁₂ ou un groupe aryle facultativement substitué ; tout groupe aryle étant un groupe phényle ou naphtyle facultativement substitué avec jusqu'à cinq groupes choisis entre des groupes halogéno, alkyle en C₁ à C₁₂, phényle, alkoxy en C₁ à C₁₂, hydroxy, amino, nitro, (alkoxy en C₁ à C₁₂)-carbonyle, (alkoxy en C₁ à C₁₂)-carbonyle-(alkyle en C₁ à C₁₂), (alkyle en C₁ à C₁₂)-carbonyloxy ou (alkyle en C₁ à C₁₂)-carbonyle.

2. Composé suivant la revendication 1, dans lequel R₁ et R₂ représentent chacun, indépendamment, l'hydrogène ou un groupe alkyle en C₁ à C₁₂.

3. Composé suivant la revendication 1 ou la revendication 2, dans lequel R₁ et R₂ représentent chacun l'hydrogène;

4. Composé suivant l'une quelconque des revendications 1 à 3, dans lequel R₃ représente un groupe benzyle.

5. Composé suivant l'une quelconque des revendications 1 à 4, dans lequel R₄ représente un groupe alkyle en C₁ à C₁₂.

6. Composé suivant la revendication 1 répondant à la formule (II), ou un de ses sels ou produits de solvatation, 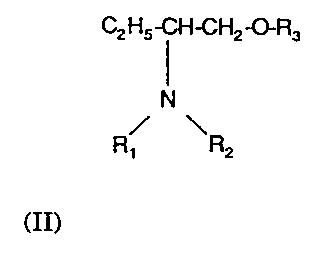 formule dans laquelle chacun des groupes R₁ et R₂ représente l'hydrogène, un groupe alkyle en C₁ à C₆, phényle facultativement substitué ou phényl-(alkyle en C₁ à C₆) facultativement substitué ; et R₃ représente un groupe phényle facultativement substitué ou phényl-(alkyle en C₁ à C₆) facultativement substitué.

7. Composé de formule (I) suivant la revendication 1, ou de ses sels ou produits de solvatation, sous forme d'un énantiomère optiquement pur.

8. Composé suivant la revendication 1, choisi dans le groupe consistant en :
le (R)-(-)-2-amino-1-(2,4-dichlorobenzyloxy)butane ;
le (R)-(-)-2-amino-1-benzyloxybutane ;
le(R)-(-)-2-amino-1-(4-chlorobenzyloxy)butane ;
le (R)-(-)-2-amino-1-(4-bromobenzyloxy)butane ; et
le(S)-(+)-2-amino-1-(4-chlorobenzyloxy)butane ; ou un de ses sels ou produits de solvatation.

9. Procédé de préparation d'un composé de formule (I) suivant la revendication 1, procédé qui comprend la réaction d'un composé de formule (IV) : 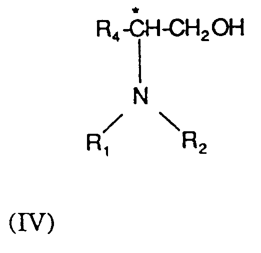 dans laquelle R₁, R₂ et R₄ répondent aux définitions mentionnées en rapport avec la formule (I), avec un composé de formule (V) :
Hal-R₃ (V)
dans laquelle Hal représente un halogène, de préférence le chlore, et R₃ répond à la définition mentionnée en rapport avec la formule (I) ; puis, si cela est requis, la préparation d'un sel ou d'un produit de solvatation du composé de formule (I).

10. Procédé pour la résolution d'un racémate acide en énantiomères distincts, procédé qui comprend :
(i) la préparation de sels diastéréoisomères à partir du racémate acide et d'un seul énantiomère d'un composé de formule (I) suivant la revendication 1 ou d'un de ses sels ou produits de solvatation ;
(ii) la séparation des sels diastéréoisomères ; et
(iii) la préparation d'un énantiomère distinct du racémate acide à partir d'un sel diastéréoisomère.

11. Procédé suivant la revendication 10, pour la préparation de la DL-N-acétyl-4-HPG ou de la DL-N-halogénacétyl-4-HPG sous une forme D(-) optiquement active, qui comprend les étapes consistant à traiter chaque composé, en solution, avec un composé de formule (I), ou de ses sels ou produits de solvatation, pour préparer le mélange de sels diastéréoisomères, à laisser le sel diastéréoisomère contenant la forme D(-) optiquement active (le sel contenant D(-)) précipité, à traiter le sel contenant D(-) avec une base, et à séparer la forme D(-) précipitée de la solution.
